(19) Europäisches Patentamt / European Patent Office / Office européen des brevets

(11) **EP 4 505 165 B1**

(12) **EUROPEAN PATENT SPECIFICATION**

(45) Date of publication and mention
of the grant of the patent:
**03.06.2026 Bulletin 2026/23**

(21) Application number: **23721464.8**

(22) Date of filing: **05.04.2023**

(51) International Patent Classification (IPC):
$G01N\ 23/087^{(2018.01)}$ $G01N\ 33/38^{(2006.01)}$

(52) Cooperative Patent Classification (CPC):
**G01N 33/389; G01N 23/087**

(86) International application number:
**PCT/IB2023/053451**

(87) International publication number:
**WO 2023/194919 (12.10.2023 Gazette 2023/41)**

(54) **METHOD FOR DIAMOND DETECTION**

VERFAHREN ZUR DIAMANTERKENNUNG

PROCÉDÉ DE DÉTECTION DE DIAMANT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **05.04.2022 AU 2022900894**

(43) Date of publication of application:
**12.02.2025 Bulletin 2025/07**

(73) Proprietors:
• **GEM RECOVERY SYSTEMS LIMITED**
London SW6 5SJ (GB)
• **Fraunhofer-Gesellschaft zur Förderung
der angewandten Forschung e.V.**
80686 München (DE)

(72) Inventors:
• **SPENCER, Roy George Stamford**
St. Albans Hertfordshire AL3 4LY (GB)
• **FIRSCHING, Markus**
90425 Nürnberg (DE)
• **ENNEN, Alexander**
90449 Nürnberg (DE)
• **LEISNER, Johannes**
90449 Fürth (DE)

(74) Representative: **HGF**
HGF Limited
4th Floor, 1 City Square
Leeds LS1 2ES (GB)

(56) References cited:
AU-B2- 689 515   RU-C1- 2 715 374
US-A- 5 603 414   US-B2- 11 073 488

• MARKUS FIRSCHING ET AL: "Detection of Enclosed Diamonds using Dual Energy X-ray imaging", 18TH WORLD CONFERENCE ON NONDESTRUCTIVE TESTING, 20 April 2012 (2012-04-20), XP055604463, Retrieved from the Internet <URL:https://www.ndt.net/article/wcndt2012/papers/397_wcndtfinal00397.pdf> [retrieved on 20190710]
• KUPTSOV V D ET AL: "The system of registration and calculation of penetrating X-ray", 2015 INTERNATIONAL SIBERIAN CONFERENCE ON CONTROL AND COMMUNICATIONS (SIBCON), IEEE, 21 May 2015 (2015-05-21), pages 1 - 5, XP033167016, DOI: 10.1109/SIBCON.2015.7147301
• SIDKY EMIL ET AL: "A robust method of x-ray source spectrum estimation from transmission measurements: Demonstrated on computer simulated, scatter-free transmission data", JOURNAL OF APPLIED PHYSICS, AMERICAN INSTITUTE OF PHYSICS, 2 HUNTINGTON QUADRANGLE, MELVILLE, NY 11747, vol. 97, no. 12, 16 June 2005 (2005-06-16), pages 124701 - 124701, XP012070434, ISSN: 0021-8979, DOI: 10.1063/1.1928312

**Description**

TECHNICAL FIELD

The present invention relates to a method for the detection of diamonds in a host material. More specifically, the method of the present invention uses x-ray transmission imaging to identify diamonds in a host material. The present invention further relates to a process for sorting host materials to produce a concentrate ore stream with an increased diamond content.

BACKGROUND ART

The following discussion of the background art is intended to facilitate an understanding of the present invention only. The discussion is not an acknowledgement or admission that any of the material referred to is or was part of the common general knowledge as at the priority date of the application.

Diamonds are naturally occurring minerals that may be found within a host material deposit. The host material may be a hard rock ore, such as kimberlite and lamproites. The ore is generally blast mined and directed to a processing plant for sorting. The ore is crushed to a relatively coarse size and is washed before sorting. The sorting process typically includes several additional crushing stages to produce a feed with a desired size. Cyclonic separation is used to separate out the heavier material which is likely to host denser diamond materials. Diamonds may also be found in alluvial deposits such as sand, gravel and clay. In such deposits, the diamonds have been separated from the hard rock host material by natural erosion. Whilst such deposits do not generally require crushing, the materials must similarly be washed and subjected to a screening and separation process. The concentrated material resulting from either deposit is subsequently subjected to a diamond detection process.

The most common method for diamond detection is with x-ray luminescence which exploits the luminescent properties of diamonds when exposed to x-rays to separate out diamond containing material. The sorting process is automated, with sensors being used to detect emitted light and separate out the diamond material. Sorting systems that rely on x-ray luminescence require the emitted light to reach the sensor. If the diamond is encased or otherwise shrouded in rock, the diamond is not detected and will not be recovered from the stream being processed. The crushing stages are used to expose the diamonds to allow for detection.

Only a small fraction of the host material contains diamonds and so a significant amount of material must be mined and processed in order for the relatively small amount of diamonds to be extracted. This requires a large sorting facility with high capital and operating costs. This cost is compounded for hard rock deposits where a significant amount of additional crushing to a small size is required. Furthermore, the crushing of large encased diamonds to smaller pieces is also undesirable as the small pieces are of lower value than the original large diamond.

Throughout this specification, unless the context requires otherwise, the word "comprise" or variations such as "comprises" or "comprising", will be understood to imply the inclusion of a stated integer or group of integers but not the exclusion of any other integer or group of integers. RU2715374 discloses X-ray separation of diamond-containing rock of different size classes.

SUMMARY OF INVENTION

In accordance with a first aspect of the present invention, there is provided a method for the identification of diamond in a host material, according to claim 1.

The inventors have found that it is possible to identify the presence of diamonds wholly encased, or otherwise shielded, by a host material by collecting x-ray attenuation data at different energy spectra. This data can be processed and combined to identify the presence of diamonds in the host material. This identification method may be used to identify the presence of diamonds in mined material. Portions of the mined material in which diamonds have been identified may then be separated to produce a concentrate.

Preferably, the host material is analysed to identify diamonds within the host material. In the case of a hard rock deposits, the analysis allows particles containing diamonds to be identified and separated for further processing. In the case of alluvial deposits, the analysis will identify diamond crystals within the heterogeneous host material.

In one form of the present invention, host material is subjected to a crushing step prior to the step of directing an x-ray beam at the host material. It would be understood that alluvial deposits may not require crushing

In one form of the present invention, x-ray attenuation data is collected at least at a low x-ray energy spectrum and a high x-ray energy spectrum.

In one form of the present invention, two or more x-rays beams at different energy spectra are directed at the host material. Preferably, the different energy spectra are achieved by varying the acceleration voltage of the x-ray tube that generates the x-ray beam or variation of the prefiltering of the x-ray beam. In one form of the present invention, the two or more beams are directed at the host material successively. Alternatively, the two or more beams are directed at the host material simultaneously. It is envisaged that this embodiment would require individual x-ray sources and x-ray detectors for each x-ray beam. It is further envisaged that the individual x-ray sources and x-ray detectors would be separated by appropriate shielding to prevent radiation interference between them.

In an alternative form of the present invention, x-ray attenuation data from a single x-ray beam is detected at multiple energy spectra. Preferably, x-ray attenuation data is detected at least at a high energy spectrum and a low energy spectrum. More preferably, an energy resolving detector is used to detect the x-rays at multiple energy spectra.

In one form of the present invention, processing the x-ray attenuation data comprises calculating the attenuated x-ray intensity as a function of a material composed of both diamond and host material. Preferably, the x-ray attenuation data is represented as a function of the thickness and attenuation coefficient of diamond and the thickness and attenuation coefficient of the host material. Alternatively, the x-ray attenuation data is represented as a function of the areal density and mass attenuation coefficient of diamond and the areal density and mass attenuation coefficient of the host material.

In one form of the present invention, combining the set of physical properties comprises the calculation of the thickness of diamond in the host material. Preferably, the calculated thickness is used to identify the presence of diamond in the host material. In an alternative form of the present invention, combining the set of physical properties comprises the calculation of the areal density of the diamond in the host material. Preferably, the calculated areal density is used to identify the presence of diamond in the host material.

According to the claimed invention, the determined physical properties at each energy spectra is offset by a calibration parameter prior to the step of combining the set of physical properties.

According to the claimed invention, the method further comprises the step of determining a calibration parameter for each energy spectra. According to the claimed invention, determining the calibration parameter comprises testing a reference sample comprising a host reference material to acquire reference x-ray attenuation data at each energy spectra; calculating theoretical x-ray attenuation data for the reference material at each energy spectra; and determining a calibration parameter for each energy spectra based on the difference between the reference x-ray attenuation data and the theoretical x-ray attenuation data. In one form of the present invention, the reference sample comprises both a host reference material and a diamond reference material.

In one form of the present invention, the effective atomic number of the host reference material ($Z_{eff\,(host\,ref)}$) is similar to the effective atomic number of the host material ($Z_{eff\,(host)}$). Suitable host reference materials include ore, aluminium, quartz or glass. The particular host reference material will depend on the particular host material of the sample. In a preferred form of the present invention, $Z_{eff\,(host\,ref)} = Z_{eff\,(host)} \pm 3$.

In one form of the present invention, the effective atomic number of the diamond reference material ($Z_{eff\,(diamond\,ref)}$) is similar to the effective atomic number of diamond ($Z_{eff\,(diamond)}$). Suitable diamond reference materials include diamond, carbon or graphite. In a preferred form of the present invention, $Z_{eff\,(diamond\,ref)} = Z_{eff\,(diamond)} \pm 1$.

In accordance with a further embodiment of the present invention, there is provided a system for the identification of diamonds in a host material feed, according to claim 11.

The system of the present invention is intended to process mined material to identify host material particles that contain diamonds and separate out the identified particles. The mined material is loaded onto the material transfer means and is passed between the one or more x-ray sources and the x-ray detection means. The x-rays emitted from the or each x-ray source will strike the host material particles and an attenuated x-ray will pass through the host material particles and will strike the x-ray detection means. The x-ray detection means will collect x-ray attenuation data of each host material particle at multiple x-ray energy spectra and will pass this data to the processing unit. The processing unit will process and combine the x-ray attenuation data to identify host material particles that contain diamond. The sorting mechanism will separate the identified host material particles that contain diamond to produce a concentrated diamond host material. The present invention can detect diamonds that are wholly encased, or otherwise shielded, by host materials. This has been found to avoid the need for fine grinding of the host material to identify diamonds. The advantages of this include a reduction in processing costs and the potential to obtain large diamonds which would otherwise be crushed in the fine grind process. The present invention can similarly detect diamonds that are contained within alluvial deposits. The x-ray beam is able to penetrate the barren host materials and identify diamond crystals within the host material.

In one form of the present invention, the sorting mechanism comprises a blow-out mechanism to selectively eject diamond containing particles from the mined material. Preferably, the sorting mechanism is in communication with the processing unit.

Preferably, the x-ray detection means detects x-ray attenuation data at least at a low x-ray energy spectrum and a high x-ray energy spectrum. In one form of the present invention, the x-ray detection means comprises two or more x-ray detectors to resolve x-ray attenuation data at separate energy spectra. Suitable detector means for use in the present invention are, for example, photodiode arrays equipped with a scintillator for converting x-rays into visible light or direct converting detectors using semiconductors as sensor layer and pixel electronics to achieve single photon counting. In an alternative form of the present invention, the x-ray detection means comprises an x-ray detector that can resolve x-ray attenuation data at multiple energy spectra. Such x-ray detectors are commonly referred to as "dual energy x-ray detectors" and comprise two detection blocks of scintillator and photodiode arrays stacked on top of each other. The first block serves as a filter for the second block, resulting in detection at different spectral sensitivities.

In one form of the present invention, the system comprises two or more x-ray sources with different emitted energy spectra. Preferably, the x-ray detection means comprises an individual x-ray detector for each x-ray source.

In an alternative form of the present invention, the system comprises one or more filters configured to be placed between the x-ray source and the x-ray detection means to provide multiple x-ray energy spectra.

Preferably, the host material is subjected to one or more pre-processing steps. More preferably, the pre-processing steps include a size reduction step and a classification step.

In one form of the present invention, the system comprises a means to distribute the host material particles on the transfer means. Preferably, a chute or vibrating feeder is used to distribute the host material particles on the transfer means.

Preferably, the transfer means is a conveyor.

DESCRIPTION OF EMBODIMENTS

A first aspect of the present invention is directed towards a method for the identification of diamonds in host materials. The present invention includes the general steps of directing x-rays at the host material; detecting x-rays passed through the host material at two or more separate energy spectra to acquire x-ray attenuation data at each energy spectra; processing the x-ray attenuation data to determine a set of physical properties of the host material at each energy spectra; and combining the set of physical properties to identify the presence of diamond in the host material.

The inventors have found that it is possible to identify the presence of diamonds wholly encased, or otherwise shielded, by host material by collecting x-ray attenuation data at different energy spectra. This data can be processed and combined to identify the presence of diamonds in the host material. This identification method may be used to identify ore particles containing diamonds in mined material to concentrate the mined material. This has been found to reduce the amount of mined material that is subsequently processed to extract the diamonds and in turn, the associated processing costs. Similarly, the present invention may be used to identify diamonds in alluvia deposits where the host material might otherwise shield the diamonds. The present invention may be used to separate out the individual diamonds from the alluvia deposit or to separate out portions of the host material containing diamonds. The following discussion is made with reference to the processing of a hard rock ore, but it should be understood that an alluvia deposit may similarly be processed.

In one embodiment, the method of the present invention is preferably used to analyse individual ore particles to identify the presence of diamond materials within the ore particle. This method requires the use of x-ray imaging techniques to collect data for this analysis. As would be appreciated by a person skilled in the art, x-ray imaging techniques generally include transmitting an x-ray beam through a target and receiving the transmitted x-rays at a detection device. As the x-ray beam travels through the target, the interaction with the target will alter the x-rays. This is referred to commonly as attenuation. The degree of attenuation is dependent on the type of material, the thickness of the material and the energy of the x-rays. The measurements made by the detector are used to measure this attenuation. Throughout this specification, unless the context requires otherwise, the term "x-ray attenuation data" is used to describe the data generated by the detector.

Essential to the method of the present invention is the capture of x-ray attenuation data at two or more energy spectra. This is commonly referred to as "dual energy x-ray transmission". This technique exploits the relative attenuation of x-rays at different energy spectra. This typically includes the separation of low energy x-ray attenuation data and high energy x-ray attenuation data. This separation can generally be achieved using two approaches. A first approach consists of directing two x-ray beams of different x-ray energy spectra at the ore. The change in the energy spectra is typically achieved through the variation of the acceleration voltage of the x-ray tube and/or with the use of filters placed between the x-ray source and the x-ray detector. A separate detector is required for each x-ray beam. The x-ray beams may be directed at the ore simultaneously or successively. If two x-ray beams at different x-ray tube voltages are used simultaneously, the beams need to be separated spatially, for example, by using radiation protection to make sure that only the x-ray beam from the first x-ray tube reaches the first detector and only the x-ray beam from the second x-ray tube reaches the second detector. Alternatively, successive x-ray attenuation data can be obtained at each x-ray energy spectra. However, this approach requires the target to be stationary or requires images to be matched based on the target movement rate.

In a second approach, the x-ray detector is adapted to separate x-ray attenuation data at different energy spectra. Such detectors typically consist of two receptor layers separated by an intermediate filter. The front detection layer records the low-energy photons, while the back detection layer records the high-energy photons. The sets of separate x-ray attenuation data at different energy spectra are acquired simultaneously. The resulting energy spectra response of such detectors looks similar to the spectra obtained using separate x-ray energy spectra as the first detection layer works as a filter for the second one. It is envisaged that any x-ray transmission and detection system known to those in the art may be utilised in the present invention.

The present invention requires that x-ray attenuation data is collected, at least, at two energy spectra. These are termed a low energy (LE) spectrum and a high energy (HE) spectrum. The measurable spectrum ($I_0(E)$) consists of two factors, the source spectrum S(E) and detector efficiency spectrum D(E). $I_0(E)$ may be changed by altering either or both of S(E) and D(E). Either of the above approaches allow for the capture of x-ray attenuation data at different energy spectra. In embodiments where two or more sources and two or more detectors are utilised, the S(E) is altered at the source, along with the freedom to change D(E) by using detectors with different spectral sensitivity. In embodiments where a single source and a dual energy (or energy sensitive) detector is utilised, only D(E) may be altered, with S(E) remaining constant.

Only the product $I_0(E)$ is relevant to the scope of this invention. One constraint is that each measurable spectrum used ($I_0(LE)$ and $I_0(HE)$) is linearly independent. In the case of two (LE and HE) they differ by more than just a constant scalar factor. One of the challenges is to find a suitable combination of LE and HE. The choice of energy spectra should aim to minimise the overlap between the two (biggest possible "difference"), whilst also ensuring that two transmission images with acceptable image quality are produced.

In one embodiment, attenuation data from at least two energy spectra levels are used having a difference in acceleration voltage of at least 150 kV. In accordance with a further preferred embodiment an energy spectrum between 150 kV and 300 kV and energy spectrum of between 300 kV and 600 kV is used.

The inventors have found that the x-ray attenuation data can be processed to calculate a series of physical material properties which can be used to usefully identify the signature of both the diamond and the host material located together in a single rock sample. The basis for this processing is the calculation of the x-ray attenuation data as a function of the attenuation coefficients and thickness of two standard materials which, when combined, produce attenuation equivalent to the object being measured.

The intensity in the x-ray attenuation data with respect to the photon energy is given by Lambert-Beer's law (1):

$$I(x,y) = \int dE\, S(E)D(E)e^{-\mu(E)d(x,y)} \quad (1)$$

In this equation, "I(x,y)" is the attenuated intensity at the spatial position "(x,y)" in the image. "E" is the photon energy, "S(E)" is the spectrum of the non-attenuated intensity from the x-ray tube (the incoming spectrum), "D(E)" is the spectral detector efficiency, "e" is Euler's constant, "$\mu$" is the attenuation coefficient of the material and "d(x,y)" is the thickness of that material at the position "(x,y)". Alternatively, this equation could also be written using the mass attenuation coefficient "$\mu$'" times the areal density "a". The following discussion is made on the basis of material thickness, but it should be understood that areal density may also be equally substituted. This equality follows directly from the definition of mass attenuation coefficient $\mu' = \mu/\rho$, where $\mu$ is the attenuation coefficient and $\rho$ is the mass density and the definition of areal density $a = d \cdot \rho$, where d is the thickness along the ray path of an object and $\rho$ is the mass density of a homogeneous object, as follows:

$$\mu' \cdot a = \frac{\mu}{\rho} \cdot d \cdot \rho = \mu \cdot d$$

The following discussion is made on the basis of material thickness, but it should be understood that areal density may also be equally substituted.

Equation 1 can be rewritten as a function of the product of S(E) and D(E):

$$I(x,y) = \int dE\, I_0(E)\, e^{-\mu(E)d(x,y)} \quad (2)$$

In this equation, "$I_0(E)$" is the product of S(E) and D(E). Only this energy spectrum is accessible by measurement with a given system setup and only this product is relevant for the method.

In the case of two materials, there are two attenuation coefficients (for the two materials), and the exponent portion of the exponential function of equation (2) changes as follows:

$$I(x,y) = \int dE\, I_0(E)\, e^{-[\mu_1(E)d_1(x,y)+\mu_2(E)d_2(x,y)]} \quad (3)$$

Where the "$\mu 1$" and "$\mu 2$" represent the attenuation coefficient of each material 1 and material 2, and "d1(x,y)" and "d2(x,y)" represent the thickness of each material 1 and material 2 at the position "(x,y)".

Using the above equation, the method of the present invention seeks to process the x-ray attenuation data at each energy spectra to calculate the intensity as function of only the unknown thickness of each diamond and host material in the ore particle.

The attenuation coefficient of each diamond and the host material is known from the literature (for example:

https://www.nist.gov/pml/xcom-photon-cross-sections-database). The x-ray properties of the source S(E) and detector D(E) are also required. It is sufficient to know the product $I_0(E)$. These x-ray spectra may be reconstructed from measurements at the detector of samples of known materials and thicknesses. A suitable method for determining such properties is published in Sidky et al (2005).

The only remaining unknown values in equation (3) are the thickness of material 1 and material 2. As equation (3) is dependent on the incoming spectrum, the attenuated x-ray data at each energy spectra will be different. Where low energy (LE) and high energy (HE) x-ray attenuation data is acquired, the difference between them being the component "$I_0$", the following equations can be separated:

$$I_{LE}(x,y) = \int dE\, I_{LE\,0}(E)\, e^{-[\mu_1(E)d_1(x,y)+\mu_2(E)d_2(x,y)]} \quad (4)$$

$$I_{HE}(x,y) = \int dE\, I_{HE\,0}(E)\, e^{-[\mu_1(E)d_1(x,y)+\mu_2(E)d_2(x,y)]} \quad (5)$$

Processing the x-ray attenuation data at different energy spectra will therefore provide a set of physical properties of the ore based on relative influence of each material at each energy spectra. Provided that the attenuation coefficients are linearly independent, which is the case for the known host materials and diamond, this set of physical properties can then be solved for d1 and d2 to provide the thickness of each material. As a general principle and when applied to diamond detection, it can be assumed "d1" to be the host material thickness and "d2" to be the diamond thickness. d1 and d2 are calculated for all pixels, resulting in images indicating the thickness of the host material (d1) and the thickness of diamond (d2). If the calculated "d2" is above a certain threshold for a certain number of adjacent pixels (e.g. larger than zero, or another value depending on the calibration), this is taken as an indication of the presence of a diamond in the sample. Other image processing methods might be used to detect the diamonds in the diamond thickness image.

The inventors have found that the identification method may be improved by conducting a calibration step. It is understood that attenuated x-ray data may be biased/altered by various effects such as, but not limited to, Compton scattering of the x-rays and electronic noise in the detector. The calibration step has been found to negate the impact of these effects at least partially. The calibration step comprises calibration of the detectors against a reference sample to determine a calibration parameter (C) which may be incorporated into equations (3) and (4) that more accurately describe the relevant attenuated x-ray energies as follows:

$$I_{LE}(x,y) = c_{LE} + \int dE\, I_{LE\,0}(E)\, e^{-[\mu_1(E)d_1(x,y)+\mu_2(E)d_2(x,y)]} \quad (6)$$

$$I_{HE}(x,y) = c_{HE} + \int dE\, I_{HE\,0}(E)\, e^{-[\mu_1(E)d_1(x,y)+\mu_2(E)d_2(x,y)]} \quad (7)$$

Where "cLE" and "cHE" are the calibration parameters for each of the LE and HE x-ray attenuation data.

To determine these calibration parameters, a reference sample having a known thickness and attenuation coefficient is tested in order to obtain LE and HE x-ray attenuation data. In one embodiment, the reference sample comprises a host reference material having a known thickness (and attenuation coefficient. The accuracy of the calibration parameters is improved by using a host reference material with similar physical properties the particular host material of the sample. Preferably, the host reference material should have an effective atomic number ($Z_{eff\,(host\,ref)}$) similar to the effective atomic number of the host material of the sample ($Z_{eff\,(host)}$). More preferably, $Z_{eff\,(host\,ref)} = Z_{eff\,(host)} \pm 3$.

In an alternative form of the present invention, the reference material comprises both a host reference material and a diamond reference material. The accuracy of the calibration parameters can be further improved by testing a reference material that also includes a diamond reference material with similar physical properties to diamond. Preferably, the diamond reference material should have an effective atomic number ($Z_{eff\,(diamond\,ref)}$) similar to the effective atomic number of diamond ($Z_{eff\,(diamond)}$). More preferably, $Z_{eff\,(diamond\,ref)} = Z_{eff\,(diamond)} \pm 1$.

The inventors have found that the effective atomic number of the respective materials is a suitable physical property that can be relied upon to ensure that the appropriate diamond reference material and host reference material is selected. The method of the present invention processes the x-ray attenuation data as a function of the attenuation coefficient, which is dependent on the atomic number of the material. As would be appreciated by a person skilled in the art, the effective atomic number is often used to characterise the interaction of photons with composite materials. Suitable diamond reference material includes diamond, carbon and graphite. Suitable host reference materials include ores, aluminium, quartz or glass. The particular host reference material used will depend on the particular host material of the sample.

For a reference sample that comprises both a host reference material and a diamond reference material, equations (6) and (7) can be solved for the calibration parameters "cLE" and "cHE" as follows:

$$c_{LE} = I_{LE}(x,y) - \int dE \, I_{LE\,0}(E) \, e^{-[\mu_3(E)d_3(x,y)+\mu_4(E)d_4(x,y)]} \quad (8)$$

$$c_{HE} = I_{HE}(x,y) - \int dE \, I_{HE\,0}(E) \, e^{-[\mu_3(E)d_3(x,y)+\mu_4(E)d_4(x,y)]} \quad (9)$$

In these equations, $I_{LE}$ and $I_{HE}$ are the LE and HE x-ray attenuation data obtained by testing the reference sample. The integral portion of the equations represents the theoretically calculated attenuation data for the reference sample comprising a host reference material with thickness "d3(x,y)" and an attenuation coefficient "$\mu$3" and a diamond reference material with thickness "d4(x,y)" and an attenuation coefficient "$\mu$4". $c_{LE}$ and $c_{HE}$ thus compensate for the difference between actual measurement and the theoretical calculation for a given material combination. For normalized intensities, i.e. all $\int dE\, I_0(E) = 1$, all intensities are identical to their respective transmissions $T$. In embodiments where the reference sample comprises only a host material, "d4(x,y)" and "$\mu$4" can be ignored.

The calibration parameters are specific to the apparatus used to obtain the attenuation data. It is envisaged that the calibration parameters might be re-calculated at regular intervals to ensure the calibration parameters remain accurate even if the x-ray properties of the x-ray source and detector change over time, which is a known aging effect.

The calculated calibration parameters can now inserted into equations (5) and (6) and are solved as in (3) and (4). The inclusion of the calibration parameters has been found to provide more accurate results for the calculated thicknesses of both the diamond crystal and the host material which in turn provides a more accurate identification and differentiation of diamond crystals wholly enclosed in the host materials.

In accordance with a further aspect of the present invention, there is provided a system for the separation of ore particles comprising diamonds from an ore stream. The system generally comprises one or more x-ray sources; an x-ray detection means adapted to detect x-ray attenuation data at multiple x-ray energy spectra; a processing unit for processing the x-ray attenuation data to identify ore particles containing diamond; a material transfer means disposed between the x-ray source and the x-ray detection means; and a sorting mechanism for separating out the identified ore particles containing diamonds.

The system of the present invention is intended to process an ore stream to identify ore particles that contain diamonds and separate out the identified particles. Similarly, the system of the present invention may process an alluvial stream to identify diamonds and separate out the identified diamonds or portions of the stream that contain diamonds. The following discussion is made with reference to the treatment of an ore stream. The ore stream is loaded onto the material transfer means and will be passed between the x-ray source and the x-ray detection means. The x-ray detection means will collect x-ray attenuation data of individual ore particles at multiple x-ray energy spectra. The processing unit will combine the x-ray attenuation data to identify ore particles that contain diamond. The sorting mechanism will separate the identified ore particles that contain diamond from the remaining ore stream to produce a concentrated diamond ore.

Below is a description of a system for the separation of ore particles comprising diamonds from an ore stream in accordance with an embodiment of the present invention.

Mined material is directed towards a commutation apparatus to reduce the particle size. The comminution apparatus may be any known to those in the art, for example a jaw crusher, cone crusher or hammer crusher. As the system of the present invention is able to identify diamonds wholly encased in host material, the particle size of the mined material does not need to be reduced to a point at which the majority of the diamond materials in the ore are exposed at the surface of the ore. The processed material is directed to a screening apparatus and any oversize material is directed back to the commutation apparatus.

In one embodiment of the present invention, the processed material has maximum particle size of 10 cm. In one embodiment of the present invention, the processed material has maximum particle size of 9 cm. In one embodiment of the present invention, the processed material has maximum particle size of 8 cm. In one embodiment of the present invention, the processed material has maximum particle size of 7 cm. In one embodiment of the present invention, the processed material has maximum particle size of 6 cm. In one embodiment of the present invention, the processed material has maximum particle size of 5 cm.

In one embodiment of the present invention, the average particle size of the processed material is between 1 cm and 10 cm. In one embodiment of the present invention, the average particle size of the processed material is between 0.5 cm and 5 cm.

The screened particles are directed to a material transfer means for analysis. In one embodiment , the material transfer means is a conveyor. The analysis of the ore particles is assisted by the even distribution of the ore particles on the conveyor. A suitable distribution apparatus may be used to improve the distribution, of the ore particles. In one embodiment, the screened ore is directed to a chute which has an outlet positioned above the conveyor. As the ore particles travel down the chute they are accelerated and this causes the individualization of the ore particles and assists in the distribution of the ore particle on the conveyor. It is envisaged that other means for the distribution of the ore particles on the conveyor may be utilised. For example, the conveyor may be vibrated.

The conveyor will direct the ore particles through an x-ray detection zone between an x-ray source and an x-ray detection means . An x-ray beam generated at the x-ray source is directed at an ore particle. The x-ray beam will travel through the ore particle and an attenuated x-ray beam is produced. The attenuated x-ray beam is transmitted to the x-ray detection means to produce x-ray attenuation data which carries information about the composition of the ore particle. In one embodiment, the ore particles are held stationary in the x-ray detection zone during the analysis. In an alternative form of the present invention, the ore particles are continuously moved through the detection zone during the analysis.

The ore particles may be fed between the x-ray source and the x-ray detection means in a horizontal transport plane, for example, over a conveyer belt. However, it is also possible for the ore particles to be supplied falling, vertically or at an angle. Preferably the velocity of the particles to be analysed is known.

The identification method used in the system of the present invention require x-ray attenuation data of the ore particle to be acquired at different energy spectra. This process is known commonly as dual energy x-ray imaging.

In one embodiment, attenuation data at least two energy spectrum levels are used having a difference in acceleration voltage of at least 150 kV. In accordance with a further preferred embodiment an energy spectrum between 150 kV and 300 kV and energy spectrum of between 300 kV and 600 kV is used.

Different apparatus for producing x-ray attenuation data at different energy spectra is known in the art and it is envisaged that any of these could be incorporated into the system of the present invention to produce the required x-ray attenuation data. The following description of suitable x-ray source and an x-ray detection means is provided for exemplification purposes.

X-ray beams are typically produced in an x-ray tube. X-ray tubes typically produce x-rays by accelerating electrons to a high velocity with a high-voltage field and colliding them with a target. The tube consists of a source of electrons, the cathode, and the target, the anode, which are enclosed in an evacuated glass tube. The voltage applied to accelerate the electrons is in the range of 30 to 600 kilovolts (kV).

X-ray detection means are used to measure the attenuated x-rays and produce a corresponding signal based on the energy of the incoming x-ray photons. This signal is amplified or converted to another form of energy, such as an electrical signal or into visible light. The x-ray detector typically comprises a sensor layer which the attenuated x-ray strike and are converted to visible light, which is detected. Suitable detector means for the use in the present invention are for example photodiode arrays equipped with a scintillator for converting X-rays into visible light or a semiconductor detection layer in which an electrical signal is directly generated by the interacting x-rays.

The system of the present invention requires x-ray attenuation data to be acquired for multiple energy spectra. This can be achieved in multiple ways.

In one embodiment the voltage applied to the x-ray source is varied. The voltage applied to the electrons in the x-ray source directly impacts the energy spectra of the x-rays. One approach for obtaining x-ray attenuation data at different x-ray energy spectra is to vary the voltage of the x-ray tube. The voltage may be continuously switched or separate x-ray tubes operating at different voltage may be used together. A suitable sensor sensitive to each energy spectra may be used.

In an alternative form of the present invention, a filter is applied to the x-ray beam. An alternative approach to provide x-rays at different energy spectra is to apply a filter to the x-ray source. The filters are preferably metal foils through which x-rays of different energy levels is transmitted. For high energy x-ray beams, a high pass filter is used which greatly attenuates low energy x-ray beams. Metal foils with a higher density class, such as copper foil, are typically used.

In an alternative form of the present invention, the x-ray detection means can detect x-rays at multiple energy spectra. In this approach, an energy resolving detector is used to simultaneously detect the x-rays at multiple energy spectra. Such detectors comprise two separate photodiode layers with an appropriate filter between the layers.

The x-ray attenuation data is communicated to a processing unit for processing the x-ray attenuation data. The processing unit will perform a computation of the x-ray attenuation data 40 in order to identify ore particles that contain diamond. The computation conducted is that as described in the first aspect of the present invention.

The processing unit will generate data which indicates or otherwise highlights the ore particles that are determined to contain diamonds. This data will provide positional data of the ore particles that are determined to contain diamonds to allow for separation. In one embodiment of the present invention, the generated data is likely to be in the form of an electronic image of the ore particles. In this embodiment, the processing unit further comprises an image processing module to analyse the produced image and to identify the ore particles that are determined to contain diamond. The position of these ore particles is used to generate positional data. The positional data is communicated to a sorting mechanism to separate the identified ore particles. In one embodiment, the sorting mechanism is a blow-out apparatus. As would be appreciated by a person skilled in the art, blow-out apparatus employ an array of air nozzles beneath the conveyor . The map data is used to trigger a blast of compressed air at individual air nozzles to change the trajectory of the identified ore particles . This will direct the identified ore particles to a concentrated material bin. The remaining ore particles are not ejected from the conveyor and are directed to a gangue material bin .

The concentrated material may then be processed in accordance with methods known in the art to extract diamonds contained within the material. As the system of the present invention is able to identify ore particles that are likely to contain diamonds, the quantity of material that require further processing is reduced. This in turn reduces the downstream processing costs. A further advantage of the system of the present invention has over sorting units that rely on x-ray luminescence is that the ore does not need to be crushed to size where diamonds are exposed at the surface. This reduces upstream processing costs to commute the material to the required size and substantially avoids the crushing of larger diamonds within the ore.

EXAMPLE 1

Example experiments were carried out using an x-ray beam with an energy spectrum of 300 kV with 2 mm aluminum filtering as low energy (LE) and 300 kV with 3 mm copper filtering as high energy (HE). Both x-ray spectra were calculated using the method discussed in [0049].

The calibration sample (the host reference material) was a block of aluminium with a thickness of 20 mm. At first, the transmission T through the calibration sample was measured. Transmission is the attenuated intensity I divided by the unattenuated intensity I0, T = I / I0

In the next step, the theoretical transmissions for that calibration sample at these spectra were calculated according to Lambert-Beers law. The results of the measured and calculated transmissions as well as the resulting calibration constant are shown in Table 1.

Table 1: Results

| Spectrum | Measured Transmission 20 mm Al | Calculated Transmission 20 mm Al | Calibration Const |
| --- | --- | --- | --- |
| LE (300 kV 2 mm Al) | T_meas_LE = 0.300300 | T_calc_LE = 0.297922 | cLE = 0.002378 |
| HE (300 kV 3 mm Cu) | T_meas_HE = 0.467964 | T_calc_HE = 0.460619 | cHE = 0.007345 |

The test sample was a kimberlite rock of approx. 30 mm thickness with several diamonds of 5-7 mm diameter. First, the basis material decomposition (BMD) with basis materials carbon (Z=6) and silicon (Z=13, this serves as a proxy for kimberlite) was conducted on the original x-ray images. In the next step, the calibration constant was subtracted and the BMD was performed on these images with otherwise identical parameters for comparison.

The contrast where the correction is applied is a lot higher, making it easier to detect the diamonds and allowing even smaller diamonds to be detected.

A measure for automatic detectability in images is the signal difference to noise ratio (SDNR). It is defined as the signal difference of the mean value of two regions divided by the square root of the sum of the variances of the regions:

$$SDNR = \frac{I_2 - I_1}{\sqrt{\sigma_1^2 + \sigma_2^2}}$$

The two different regions (just kimberlite and diamond in kimberlite) carry the index 1 (just kimberlite) and 2 (kimberlite and diamond).

From those line profiles, SDNR was calculated, the region 1 being the region with the diamond (from pixel 90 to 120) and the region 2 being the region without diamond (from pixel 1 to 80). The resulting SDNR is 1.768 for the uncorrected image and 3.563 for the corrected one, which shows the significant increase of the diamond detection capability of the method of the present invention.

**Claims**

**1.** A method for the identification of diamond in a host material, the method comprising the steps of:

directing an x-ray beam at the host material;
detecting x-rays passed through the host material at two or more separate energy spectra to acquire x-ray attenuation data at each energy spectra;
processing the x-ray attenuation data to determine a set of physical properties of the host material at each energy spectra;
offsetting the determined physical properties at each energy spectra by a calibration parameter; and
combining the set of physical properties to identify the presence of diamond in the host material,

wherein the calculation of the calibration parameter comprises:

testing a reference sample comprising a host reference material to acquire reference x-ray attenuation data at each energy spectra;

calculating theoretical x-ray attenuation data for the reference material at each energy spectra; and

determining a calibration parameter for each energy spectra based on the difference between the reference x-ray attenuation data and the theoretical x-ray attenuation data.

2. A method according to claim 1, wherein x-ray attenuation data is collected at least at a low x-ray energy spectrum and a high x-ray energy spectrum.

3. A method according to claim 2, wherein two or more x-rays beams at different energy spectra are directed at the host material.

4. A method according to claim 3, wherein the two or more beams are directed at the host material successively or wherein the two or more beams are directed at the host material simultaneously.

5. A method according to claim 2, wherein x-ray attenuation data from a single x-ray beam is detected at multiple energy spectra.

6. A method according to claim 5, wherein an energy resolving detector is used to detect the x-rays at multiple energy spectra.

7. A method according to any one of the preceding claims, wherein processing the x-ray attenuation data comprises calculating the attenuated x-ray intensity as a function of a material composed of both diamond and host material.

8. A method according to claim 7, wherein the x-ray attenuation data is represented as a function of the thickness and attenuation coefficient of diamond and the thickness and attenuation coefficient of the host material, preferably wherein combining the set of physical properties comprises the calculation of the thickness of diamond in the host material.

9. A method according to claim 7, wherein the x-ray attenuation data is represented as a function of the areal density and mass attenuation coefficient of diamond and the areal density and mass attenuation coefficient of the host material, preferably wherein combining the set of physical properties comprises the calculation of the areal density of the diamond in the host material.

10. A method according to any one of the preceding claims, wherein the reference sample comprises both a host reference material and a diamond reference material.

11. A system for the identification of diamonds in an ore host material feed, the system comprising:

one or more x-ray sources;
a x-ray detection means adapted to detect x-ray attenuation data at multiple x-ray energy spectra;
a processing unit for processing the x-ray attenuation data according to the method of any of claims 1 to 14 to identify host material particles containing diamond;
a material transfer means disposed between the x-ray source and the x-ray detection means; and
a sorting mechanism for separating out the identified host material particles containing diamonds.

12. A system according to claim 11, wherein the x-ray detection means comprises two or more x-ray detectors to resolve x-ray attenuation data at separate energy spectra.

13. A system according to claim 11, wherein the x-ray detection means comprises an x-ray detector that can resolve x-ray attenuation data at multiple energy spectra.

14. A system according to any one of claims 11 to 13, wherein the sorting mechanism comprises a blow-out mechanism to selectively eject diamond containing particles from the mined material.

15. A system according to any one of claims 11 to 14, wherein the system comprises a means to distribute the host material particles on the transfer means, preferably wherein a chute or vibrating feeder is used to distribute the host material particles on the transfer means.

**Patentansprüche**

1. Verfahren für die Identifizierung von Diamant in einem Wirtsmaterial, wobei das Verfahren die folgenden Schritte umfasst:

   Richten eines Röntgenstrahlbündels auf das Wirtsmaterial;
   Erkennen von Röntgenstrahlen, die bei zwei oder mehr separaten Energiespektren durch das Wirtsmaterial geleitet werden, um Röntgenstrahldämpfungsdaten bei jedem Energiespektrum zu erfassen;
   Verarbeiten der Röntgenstrahldämpfungsdaten, um einen Satz von physikalischen Eigenschaften des Wirtsmaterials bei jedem Energiespektrum zu bestimmen;
   Kompensieren der bestimmten physikalischen Eigenschaften bei jedem Energiespektrum durch einen Kalibrierungsparameter; und
   Kombinieren des Satzes von physikalischen Eigenschaften, um das Vorhandensein von Diamant in dem Wirtsmaterial zu identifizieren,
   wobei die Berechnung des Kalibrierungsparameters Folgendes umfasst:

   Testen einer Referenzprobe, die ein Wirtsreferenzmaterial umfasst, um Referenzröntgenstrahldämpfungsdaten bei jedem Energiespektrum zu erfassen;
   Berechnen der theoretischen Röntgenstrahldämpfungsdaten für das Referenzmaterial bei jedem Energiespektrum; und
   Bestimmen eines Kalibrierungsparameters für jedes Energiespektrum basierend auf der Differenz zwischen den Referenzröntgenstrahldämpfungsdaten und den theoretischen Röntgenstrahldämpfungsdaten.

2. Verfahren nach Anspruch 1, wobei Röntgenstrahldämpfungsdaten mindestens bei einem niedrigen Röntgenstrahlenergiespektrum und einem hohen Röntgenstrahlenergiespektrum gesammelt werden.

3. Verfahren nach Anspruch 2, wobei zwei oder mehr Röntgenstrahlen mit unterschiedlichen Energiespektren auf das Wirtsmaterial gerichtet werden.

4. Verfahren nach Anspruch 3, wobei die zwei oder mehr Strahlen nacheinander auf das Wirtsmaterial gerichtet werden oder
   wobei die zwei oder mehr Strahlen gleichzeitig auf das Wirtsmaterial gerichtet werden.

5. Verfahren nach Anspruch 2, wobei Röntgenstrahldämpfungsdaten von einem einzelnen Röntgenstrahlbündel bei mehreren Energiespektren erkannt werden.

6. Verfahren nach Anspruch 5, wobei eine energieauflösende Erkennungseinrichtung verwendet wird, um die Röntgenstrahlen bei mehreren Energiespektren zu erkennen.

7. Verfahren nach einem der vorhergehenden Ansprüche, wobei das Verarbeiten der Röntgenstrahldämpfungsdaten Berechnen der gedämpften Röntgenstrahlintensität in Abhängigkeit von einem Material umfasst, das sowohl aus Diamant als auch aus Wirtsmaterial zusammengesetzt ist.

8. Verfahren nach Anspruch 7, wobei die Röntgenstrahldämpfungsdaten in Abhängigkeit von der Dicke und des Dämpfungskoeffizienten von Diamant und der Dicke und des Dämpfungskoeffizienten des Wirtsmaterials dargestellt werden,
   wobei das Kombinieren des Satzes von physikalischen Eigenschaften vorzugsweise die Berechnung der Dicke von Diamant in dem Wirtsmaterial umfasst.

9. Verfahren nach Anspruch 7, wobei die Röntgenstrahldämpfungsdaten in Abhängigkeit von der Flächendichte und des Massendämpfungskoeffizienten von Diamant und der Flächendichte und des Massendämpfungskoeffizienten des Wirtsmaterials dargestellt werden,
   wobei das Kombinieren des Satzes von physikalischen Eigenschaften vorzugsweise die Berechnung der Flächendichte des Diamanten in dem Wirtsmaterial umfasst.

10. Verfahren nach einem der vorhergehenden Ansprüche, wobei die Referenzprobe sowohl ein Wirtsreferenzmaterial als auch ein Diamantreferenzmaterial umfasst.

11. System für die Identifizierung von Diamanten in einer Erzwirtsmaterialzufuhr, wobei das System Folgendes umfasst:

eine oder mehrere Röntgenstrahlquellen;

ein Röntgenstrahlerkennungsmittel, das dazu ausgelegt ist, Röntgenstrahldämpfungsdaten bei mehreren Röntgenstrahlenergiespektren zu erkennen;

eine Verarbeitungseinheit zum Verarbeiten der Röntgenstrahldämpfungsdaten gemäß dem Verfahren nach einem der Ansprüche 1 bis 14, um Wirtsmaterialpartikel, die Diamant enthalten, zu identifizieren;

ein Materialübertragungsmittel, das zwischen der Röntgenstrahlquelle und dem Röntgenstrahlerkennungsmittel angeordnet ist; und

einen Sortiermechanismus zum Abtrennen der identifizierten Wirtsmaterialpartikel, die Diamanten enthalten.

12. System nach Anspruch 11, wobei das Röntgenstrahlerkennungsmittel zwei oder mehr Röntgenstrahlenerkennungseinrichtungen umfasst, um Röntgenstrahldämpfungsdaten bei separaten Energiespektren aufzulösen.

13. System nach Anspruch 11, wobei das Röntgenstrahlerkennungsmittel eine Röntgenstrahlerkennungseinrichtung umfasst, die Röntgenstrahldämpfungsdaten bei mehreren Energiespektren auflösen kann.

14. System nach einem der Ansprüche 11 bis 13, wobei der Sortiermechanismus einen Ausblasmechanismus zum selektiven Ausstoßen von diamanthaltigen Partikeln aus dem abgebauten Material umfasst.

15. System nach einem der Ansprüche 11 bis 14, wobei das System ein Mittel umfasst, um die Wirtsmaterialpartikel auf dem Übertragungsmittel zu verteilen,

wobei vorzugsweise eine Rutsche oder Vibrationszufuhreinrichtung verwendet wird, um die Wirtsmaterialpartikel auf dem Übertragungsmittel zu verteilen.

**Revendications**

1. Procédé permettant l'identification de diamant dans un matériau hôte, le procédé comprenant les étapes de :

direction d'un faisceau de rayons X vers le matériau hôte ;

détection des rayons X passés à travers le matériau hôte à deux spectres d'énergie séparés, ou plus, pour acquérir des données d'atténuation de rayons X à chaque spectres d'énergie ;

traitement des données d'atténuation de rayons X pour déterminer un ensemble de propriétés physiques du matériau hôte à chaque spectres d'énergie ;

décalage des propriétés physiques déterminées à chaque spectres d'énergie par un paramètre d'étalonnage ; et

la combinaison de l'ensemble des propriétés physiques pour identifier la présence de diamant dans le matériau hôte,

ledit calcul du paramètre d'étalonnage comprenant :

la mise à l'essai d'un échantillon de référence comprenant un matériau de référence hôte pour acquérir des données d'atténuation de rayons X de référence à chaque spectres d'énergie ;

calcul des données d'atténuation de rayons X théoriques pour le matériau de référence à chaque spectres d'énergie ; et

détermination d'un paramètre d'étalonnage pour chaque spectres d'énergie sur la base de la différence entre les données d'atténuation de rayons X de référence et les données d'atténuation de rayons X théoriques.

2. Procédé selon la revendication 1, des données d'atténuation de rayons X étant collectées au moins à un spectre d'énergie de rayons X faible et à un spectre d'énergie de rayons X élevée.

3. Procédé selon la revendication 2, deux faisceaux de rayons X, ou plus, à des spectres d'énergie différents étant dirigés vers le matériau hôte.

4. Procédé selon la revendication 3, lesdits deux faisceaux, ou plus, étant dirigés vers le matériau hôte successivement ou

lesdits deux faisceaux, ou plus, étant dirigés simultanément vers le matériau hôte.

5. Procédé selon la revendication 2, des données d'atténuation de rayons X provenant d'un seul faisceau de rayons X étant détectées à des spectres d'énergie multiples.

**6.** Procédé selon la revendication 5, un détecteur de résolution d'énergie étant utilisé pour détecter les rayons X à des spectres d'énergie multiples.

**7.** Procédé selon l'une quelconque des revendications précédentes, ledit traitement des données d'atténuation de rayons X comprenant le calcul de l'intensité de rayons X atténuée en fonction d'un matériau composé à la fois de diamant et de matériau hôte.

**8.** Procédé selon la revendication 7, lesdites données d'atténuation de rayons X étant représentées en fonction de l'épaisseur et du coefficient d'atténuation du diamant et de l'épaisseur et du coefficient d'atténuation du matériau hôte, de préférence ladite combinaison de l'ensemble de propriétés physiques comprenant le calcul de l'épaisseur de diamant dans le matériau hôte.

**9.** Procédé selon la revendication 7, lesdites données d'atténuation de rayons X étant représentées en fonction de la densité surfacique et du coefficient d'atténuation de masse du diamant et de la densité surfacique et du coefficient d'atténuation de masse du matériau hôte, de préférence ladite combinaison de l'ensemble de propriétés physiques comprenant le calcul de la densité surfacique du diamant dans le matériau hôte.

**10.** Procédé selon l'une quelconque des revendications précédentes, ledit échantillon de référence comprenant à la fois un matériau de référence hôte et un matériau de référence diamant.

**11.** Système pour l'identification de diamants dans une alimentation en matériau hôte de minerai, le système comprenant :

une ou plusieurs sources de rayons X ;
un moyen de détection de rayons X adapté pour détecter des données d'atténuation de rayons X à de multiples spectres d'énergie de rayons X ;
une unité de traitement destinée à traiter les données d'atténuation de rayons X selon le procédé de l'une quelconque des revendications 1 à 14 pour identifier des particules de matériau hôte contenant du diamant ;
un moyen de transfert de matériau disposé entre la source de rayons X et le moyen de détection de rayons X ; et
un mécanisme de tri destiné à séparer les particules de matériau hôte identifiées contenant des diamants.

**12.** Système selon la revendication 11, ledit moyen de détection de rayons X comprenant deux détecteurs de rayons X, ou plus, pour résoudre des données d'atténuation de rayons X à des spectres d'énergie séparés.

**13.** Système selon la revendication 11, ledit moyen de détection de rayons X comprenant un détecteur de rayons X qui peut résoudre des données d'atténuation de rayons X à des spectres d'énergie multiples.

**14.** Système selon l'une quelconque des revendications 11 à 13, ledit mécanisme de tri comprenant un mécanisme de soufflage pour éjecter sélectivement des particules contenant du diamant du matériau extrait.

**15.** Système selon l'une quelconque des revendications 11 à 14, ledit système comprenant un moyen pour distribuer les particules de matériau hôte sur le moyen de transfert, de préférence une goulotte ou un dispositif d'alimentation vibrant étant utilisé pour distribuer les particules de matériau hôte sur le moyen de transfert.

**EP 4 505 165 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2715374